Europäisches Patentamt

European Patent Office

Office européen des brevets

(11) Veröffentlichungsnummer : **0 280 211 B1**

(12) **EUROPÄISCHE PATENTSCHRIFT**

(19)

(45) Veröffentlichungstag der Patentschrift :
**02.11.94 Patentblatt 94/44**

(21) Anmeldenummer : **88102458.2**

(22) Anmeldetag : **19.02.88**

(51) Int. Cl.⁵ : **G01N 33/543,** // G01N33/537,
G01N33/576

(54) **Verfahren zur Bestimmung von Antikörpern.**

(30) Priorität : **23.02.87 DE 3705686**

(43) Veröffentlichungstag der Anmeldung :
**31.08.88 Patentblatt 88/35**

(45) Bekanntmachung des Hinweises auf die
Patenterteilung :
**02.11.94 Patentblatt 94/44**

(84) Benannte Vertragsstaaten :
**AT BE CH DE ES FR GB GR IT LI LU NL SE**

(56) Entgegenhaltungen :
**EP-A- 0 147 848
EP-A- 0 160 900
EP-A- 0 177 191
EP-A- 0 201 079
EP-A- 0 245 926
DE-A- 3 605 695
GB-A- 2 084 317**

(73) Patentinhaber : **BOEHRINGER MANNHEIM
GMBH
D-68298 Mannheim (DE)**

(72) Erfinder : **Geiger Thomas, Dr.
Iglauerstrasse 25
D-8918 Diessen (DE)**
Erfinder : **Engel, Wolf Dieter, Dr.
Moosstrasse 7
D-8121 Pähl (DE)**

(74) Vertreter : **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Bestimmung eines Antikörpers durch Inkubation mit drei verschiedenen Rezeptoren $R_1$, $R_2$ und $R_3$, von denen $R_1$ und $R_3$ in flüssiger Phase vorliegen und mit dem Antikörper bindefähig sind, $R_2$ in fester Phase vorliegt und mit $R_1$ bindefähig ist und $R_3$ eine Markierung trägt, Trennung der festen von der flüssigen Phase und Messung der Markierung in der festen Phase.

Antikörper sind Proteinmoleküle, die nach Kontakt mit dem passenden Antigen von B-Lymphocyten und Plasmazellen produziert werden und spezifisch gegen das ihre Bildung auslösende Antigen gerichtet sind. Der Nachweis von Antikörpern gegen bestimmte Antigene kann daher Aufschluß geben über das Stadium einer Krankheit oder über das Vorliegen von Autoimmunkrankheiten.

Antikörper können empfindlich nachgewiesen werden durch Verfahren nach dem Prinzip des Immunoassays. Hier sind verschiedene Varianten möglich. So kann beispielsweise das Antigen kovalent an einen Träger gebunden werden, mit der Lösung, die den zu bestimmenden Antikörper enthält, in Kontakt gebracht werden und anschließend mit einem gegen diesen Antikörper gerichteten Antikörper, in der Regel ein Anti-Ig-Antikörper, der in bekannter Weise markiert ist, umgesetzt werden. Das Maß an gebundener Markierung gibt dann Rückschluß auf die Menge an dem Antikörpergehalt. Nachteil dieses Verfahrens ist es, daß durch unspezifische Bindung von in der Probe enthaltenem, nicht spezifischem Antikörper falsch positive Werte erhalten werden. Außerdem kann diese Bestimmung nur in zwei aufeinanderfolgenden Schritten durchgeführt werden. Ein weiterer Test zum Nachweis und zur quantitativen Bestimmung eines Antikörpers besteht darin, Antigen gegen den zu bestimmenden Antikörper an einer Festphase zu fixieren. Anschließend gibt man Patientenserum zusammen mit einer vorbestimmten Menge des zu bestimmenden Antikörpers, der jedoch eine Markierung trägt, zu und mißt anschließend die an der Festphase gebundene Markierung. Es handelt sich hierbei also um einen kompetitiven Test, dessen Empfindlichkeit zu wünschen übrig läßt. Eine weitere Möglichkeit besteht darin, an eine Festphase einen Anti-Ig-Antikörper zu binden und dann mit der Testlösung umzusetzen. Anschließend wird ein für den zu bestimmenden Antikörper spezifisches Antigen, an das eine Markierung gebunden ist, zugegeben. Nachteil dieser Methode ist die begrenzte Bindekapazität der Festphase, da außer dem zu bestimmenden Antikörper auch andere Antikörper derselben Globulinklasse gebunden werden.

Eine weitere Variante besteht darin, eine Festphase mit dem spezifischen Antigen des zu bestimmenden Antikörpers zu beschichten, anschließend diese Festphase mit der Probelösung umzusetzen und danach in einem weiteren Schritt eine spezifisch bindefähige Substanz, die eine Markierung trägt, zuzusetzen. Derartige Verfahren sind beispielsweise bekannt aus EP-A 168 689 und EP-A 160 900. Nachteil dieser Verfahren ist es zum einen, daß für jede Bestimmung eine andere Festphase verwendet werden muß, da die Festphase jeweils das spezifische Antigen trägt. Ein weiterer Nachteil ist es, daß die Umsetzung des Antikörpers mit dem Antigen heterogen erfolgt. Darüberhinaus sind für Verfahren dieser Art immer zwei Schritte notwendig. Damit sind aber solche Verfahren nicht übertragbar auf Analysatoren, bei denen nur ein einziger Waschschritt durchführbar ist.

EP-A-0 201 079 beschreibt in allgemeiner Form ein immunologisches Verfahren, das sowohl zur Bestimmung von Antikörpern als auch von Antigenen geeignet sein soll. Im Gegensatz zum Verfahren der Erfindung reagieren jedoch der erste und zweite Bindungspartner mit verschiedenen Regionen des zu bestimmenden Antikörpers. Im übrigen wird im Verfahren gemäß EP-A-0 201 079 als Rezeptor $R_3$ ein markierter Antikörper gegen den zu bestimmenden Antikörper verwendet.

DE-A-36 05 695 beschreibt ebenfalls ein immunologisches Verfahren, im Rahmen dessen, wenn Antikörper analysiert werden sollen, ein erstes Antikörperreagenz durch einen Antikörper gebildet wird, der $F_c$- Fragmente aufweist, welche eine spezifische Bindung mit Substanzen 12 eines sensibilisierten unlöslichen Trägers 10 eingehen. Über die Zusammensetzung eines markierten Bindungspartners für den Analyten im Falle einer Antikörperbestimmung wird nichts ausgesagt.

Schließlich beschreibt EP-A-0 177 191 ein Analyseverfahren, bei dem eine Probe (a), bei der es sich auch um Immunoglobuline handeln kann, ein markierter Bindungspartner (b) für die Probe und ein Probenanaloges (c) als Komponenten vorgesehen sind.

Aufgabe der Erfindung war es daher, ein Verfahren zur Bestimmung von Antikörper zu schaffen, das einstufig und mit nur einem einzigen Waschschritt durchgeführt werden kann und somit automatisierbar ist. Darüberhinaus war es Aufgabe der Erfindung, ein Verfahren zu schaffen, bei dem eine feste Phase für viele verschiedene Tests verwendet werden kann.

Diese Aufgabe wird gelöst durch ein Verfahren zur Bestimmung eines Antikörpers durch Inkubation mit drei verschiedenen Rezeptoren $R_1$, $R_2$ und $R_3$, von denen $R_1$ und $R_3$ in flüssiger Phase vorliegen und mit dem Antikörper bindefähig sind, $R_2$ an eine feste Phase gebunden vorliegt und mit $R_1$ bindefähig ist und $R_3$ eine Markierung trägt, Trennung der festen von der flüssigen Phase und Messung der Markierung in der festen Phase, das dadurch gekennzeichnet ist, daß man als $R_1$ ein Konjugat aus einer vom zu bestimmenden Antikörper spezifisch erkannten Substanz und einem Reaktionspartner eines spezifischen Bindungssystems, als $R_3$ ein Konjugat aus einer vom zu bestimmenden Antikörper spezifisch erkannten Substanz und einer Markierung und als $R_2$ den anderen Bindungspartner des spezifischen Bindungssystems verwendet, wobei $R_1$ und $R_3$ jeweils das für den zu bestimmenden Antikörper spezifische Epitop tragen.

Das erfindungsgemäße Verfahren beruht auf dem Prinzip, daß zwei lösliche Bindungskomponenten, von denen die eine ein Konjugat aus einer Substanz, die mindestens das für den zu bestimmenden Antikörper spezifische Epitop trägt, wie z. B. ein Antigen, Antigenfragment, Anti-idiotyp-Antikörper oder Hapten mit einem Partner eines spezifischen Bindungssystems und die andere ein Konjugat aus einer Substanz, die das gleiche für den zu bestimmenden Antikörper spezifische Epitop trägt, wie z. B. ein Antigen, Antigenfragment, Anti-idiotyp-Antikörper oder Hapten und einer Markierung ist, um die mindestens zwei spezifisch bindefähigen Paratope des zu bestimmenden Antikörpers konkurrieren. Dabei entstehen die folgenden Reaktionsprodukte: Antikörper, die sowohl $R_1$ als auch $R_3$ gebunden haben, Antikörper, die nur $R_1$ gebunden haben und Antikörper, die nur $R_3$ gebunden haben. Nur die ersten beiden Reaktionsprodukte können an der Festphase gebunden werden, da sie einen Reaktionspartner des spezifischen Bindungssystemes tragen. Das Reaktionsprodukt, bei dem nur $R_3$ gebunden ist, wird ausgewaschen. Da der Antikörper, an den nur $R_1$ gebunden ist, keine Markierung hat, geht er in die Messung nicht ein. Die Konzentrationsbestimmung des Antikörpers erfolgt daher allein über die Antikörpermoleküle, die $R_1$ und $R_3$ gebunden haben. Dabei wird die höchste Empfindlichkeit dann erreicht, wenn die Menge an vom zu bestimmenden Antikörper spezifisch erkannter Substanz, auch als immunreaktiv bezeichnet, in $R_1$ größer oder gleich der Menge an immunreaktiver Substanz in $R_3$ ist. Bevorzugt werden die immunreaktiven Substanzen in $R_1$ und $R_3$ im Verhältnis 20 bis 1:1 eingesetzt. Besonders bevorzugt beträgt das Verhältnis 5 bis 1:1. Die Auswertung ist über das gebundene markierte Konjugat möglich, und mit Hilfe einer Eichkurve kann dann die Konzentration des Antikörpers in der Probe bestimmt werden.

Überraschenderweise ist es somit erfindungsgemäß möglich, Antikörper mit sehr hoher Genauigkeit und in einem einfachen Verfahren zu bestimmen. Das erfindungsgemäße Bestimmungsverfahren wird nicht durch hohe Konzentrationen von nicht zu bestimmenden Antikörpern gestört. Besonderer Vorteil des Verfahrens ist die Schnelligkeit und Einfachheit des Testablaufs.

Das erfindungsgemäße Verfahren ist geeignet zur Bestimmung von Antikörpern aller Klassen, wie z.B. IgG, IgM, IgA, IgD und IgE sowie Gemischen hiervon.

Da jeder der Rezeptoren und auch der zu bestimmende Antikörper jeweils nur spezifisch mit dem für ihn bestimmten Reaktionspartner reagieren kann, ist es möglich, alle Rezeptoren und die Probe zusammen zu inkubieren und das Verfahren einstufig durchzuführen. Dies erfordert dann auch nur einen einzigen Waschschritt nach der Inkubation. Selbstverständlich ist es auch möglich, das erfindungsgemäße Verfahren in zwei Stufen durchzuführen. In diesem Fall wird zweckmäßig die Probe zunächst mit den Rezeptoren $R_1$ und $R_3$ inkubiert und anschließend der aus $R_1$, $R_3$ und zu bestimmendem Antikörper entstandene Komplex zu dem festphasengebundenen $R_2$ gegeben. Ein weiterer Vorteil des erfindungsgemäßen Verfahrens liegt in der einstufigen Verfahrensweise, da es dadurch möglich ist, das Verfahren leicht zu automatisieren.

Zur Durchführung des erfindungsgemäßen Verfahrens werden drei verschiedene Rezeptoren $R_1$, $R_2$ und $R_3$ verwendet. Die Rezeptoren $R_1$ und $R_3$ liegen jeweils in flüssiger Phase und vorzugsweise in gleicher Konzentration vor. Sie bestehen jeweils aus einer Substanz, die das für den zu bestimmenden Antikörper spezifisches Epitop trägt. Diese Substanz ist jeweils ein Konjugat aus zwei Teilen, wobei der eine Teil das Epitop trägt und beispielsweise ein Antigen, ein Antigenfragment ein Anti-idiotyp-Antikörper oder ein Hapten ist. Die in den Rezeptoren $R_1$ und $R_3$ enthaltenen antikörperspezifischen Substanzen können identisch oder verschieden sein. Der andere Teil des Konjugates ist bei $R_1$ ein Reaktionspartner eines spezifischen Bindungssystemes. Unter einem spezifischen Bindungssystem werden hier zwei Partner verstanden, die spezifisch miteinander reagieren können. Das Bindungsvermögen kann dabei auf einer immunologischen Reaktion oder aber auf einer anderen spezifischen Reaktion beruhen.

Bevorzugt wird als spezifisches Bindungssystem eine Kombination von Biotin und Avidin; Biotin und Streptavidin; Biotin und Antibiotin; Hapten und Antihapten; Fcγ-Fragment eines Antikörpers und Antikörper gegen dieses Fcγ-Fragment oder Kohlehydrat und Lectin verwendet. Einer der Reaktionspartner dieses spezifisch bindefähigen Paares ist dann Teil des Konjugates, das den Rezeptor $R_1$ bildet.

Der andere Reaktionspartner ist der Rezeptor $R_2$, der in fester Phase vorliegt. Die Bindung von $R_2$ an ein unlösliches Trägermaterial kann nach den üblichen, dem Fachmann bekannten Methoden zur Fixierung von Bindungspartnern, vorzugsweise biologischer Bindungssysteme, an feste Trägersubstanzen vorgenommen werden. Sowohl eine kovalente, als auch eine adsorptive Bindung ist geeignet. Bevorzugt wird jedoch, wegen der hierbei erzielbaren höheren Ausbeute und der vereinfachten Arbeitsweise, eine lediglich adsorptive Bindung, beispielsweise an Kunststoff. Als feste Phase besonders geeignet sind Reagenzgläschen oder Mikrotiterplatten aus Polystyrol und ähnlichen Kunststoffen, die adsorptiv an der Innenoberfläche mit $R_2$ beschichtet sind. Weiterhin geeignet sind auch teilchenförmige Substanzen, z. B. Molekularsiebmaterialien, Glaskörperchen, Kunststoffschläuche und dergleichen. Besonders bevorzugt wird als Träger der festen Phase ein poröser, schichtförmiger Träger wie Papier verwendet. Als $R_2$ wird an der festen Phase der Reaktionspartner des spezifischen Bindungssystems fixiert.

Der Rezeptor $R_3$ trägt außer dem Antigen, Antigenfragment oder Hapten als anderen Teil des Konjugates eine Markierung. Bevorzugt wird als Markierung ein Enzym, eine fluoreszierende, chemilumineszierende oder radioaktive Substanz verwendet. Verfahren zur Markierung von Antigenen, Antigenfragmenten oder Haptenen sind dem Fachmann bekannt, z. B. aus Clin. Chim. Acta 81 (1977) 1 bis 40, und bedürfen hier keiner weiteren Erläuterung. Die Markierung kann in an sich bekannter Weise bestimmt werden.

In einer bevorzugten Ausführungsform des erfindungsgemäßen Verfahrens werden die Rezeptoren $R_1$ und $R_3$ sowie die den zu bestimmenden Antikörper enthaltende Probe inkubiert. Dabei reagieren $R_1$ und $R_3$ mit statistischer Wahrscheinlichkeit mit den Paratopen des zu bestimmenden Antikörpers. Diese Reagenzmischung wird anschließend in Kontakt mit der den Rezeptor $R_2$ tragenden Festphase gebracht.

Der in $R_1$ enthaltene Reaktionspartner des spezifischen Bindungssystemes reagiert mit $R_2$. Nach der Inkubation wird eine Phasentrennung der festen von der flüssigen Phase durchgeführt, beispielsweise durch Absaugen. Nach Waschen der festen Phase kann dann die Messung der Markierung vorgenommen werden und stellt ein Maß für die Menge an zu bestimmendem Antikörper dar. Erfolgt die Markierung mit einem Enzym, genügt es, einfach die Aktivität des Markierungsenzyms in der hierfür allgemein bekannten Weise zu messen. Als Markierungsenzym wird besonders bevorzugt Peroxidase oder β-Galactosidase verwendet.

Falls die Markierung nicht mit einem Enzym sondern mit einer radioaktiven Substanz, einem Isotop, einer fluoreszierenden oder chemilumineszierenden Substanz erfolgt, so wird auch hier die Bestimmung nach einer der dem Fachmann wohlbekannten Methoden vorgenommen.

Wird das Verfahren in einem Zentrifugalanalysator durchgeführt, so ist es bevorzugt, daß die Probe zuerst über einen Träger zentrifugiert wird, auf dem die beiden Rezeptoren $R_1$ und $R_3$ ablösbar vorliegen. Hierbei werden die Rezeptoren $R_1$ und $R_3$ gelöst.

Durch entsprechend zeitlich gestaltete Haltephasen wird die Ablösung der Rezeptoren $R_1$ und $R_3$ und deren Komplexbildung mit dem in der Probe enthaltenen, zu bestimmenden Antikörper bewirkt.

Im nächsten Schritt wird das gebildete Reaktionsgemisch auf die feste Phase gebracht, wo dann die endgültige Reaktion abläuft.

Ebenso ist es auch möglich, die Rezeptoren $R_1$ und $R_3$ und die Probe ohne Haltephase für die Komplexbildung direkt auf die feste Phase mit dem Rezeptor $R_2$ zu bringen. Da die Reaktion zwischen den Rezeptoren $R_1$ und $R_3$ und dem zu bestimmenden Antikörper homogen ist, läuft sie viel schneller ab als die Reaktion zwischen $R_1$ und $R_2$ an der heterogenen Phasengrenze. Diese Reaktionsführung führt daher zu Ergebnissen, die sich nicht wesentlich von denen unterscheiden, die man in der Reaktionsführung mit Haltephase erhält.

In einer besonders bevorzugten Ausführungsform des erfindungsgemäßen Verfahrens werden alle drei Rezeptoren sowie die den zu bestimmenden Antikörper enthaltende Probe inkubiert. Dabei reagieren $R_1$ und $R_3$ mit statistischer Wahrscheinlichkeit mit den Paratopen des zu bestimmenden Antikörpers. Der in $R_1$ enthaltene Reaktionspartner des spezifischen Bindungssystemes reagiert mit $R_2$. Wie zuvor beschrieben, läuft auch hier die homogene Reaktion zwischen $R_1$, $R_3$ und zu bestimmendem Antikörper wesentlich schneller ab als die heterogene Reaktion zwischen $R_1$ und $R_2$ und wird daher nicht behindert. Nach der Inkubation wird eine Phasentrennung der festen von der flüssigen Phase durchgeführt, beispielsweise durch Absaugen. Nach Waschen der festen Phase kann dann die Messung der Markierung vorgenommen werden und stellt ein Maß für die Menge an zu bestimmendem Antikörper dar.

In einer weiteren Ausführungsform ist es auch möglich, zuerst die Probe mit der festen Phase in Kontakt zu bringen und dann das Reaktionsgemisch aus den Rezeptoren $R_1$ und $R_3$ zuzugeben. Dieses Verfahren ist besonders vorteilhaft für Analysenautomaten, die Probe und Reagenz nicht gleichzeitig dosieren können.

Ein weiterer Gegenstand der Erfindung ist ein Reagenz zur Bestimmung eines Antikörpers, das dadurch gekennzeichnet ist, daß es zwei verschiedene lösliche, mit dem Antikörper bindefähige Rezeptoren $R_1$ und $R_3$, von denen $R_1$ einen Reaktionspartner eines spezifischen Bindungssystems und $R_3$ eine Markierung trägt und außerdem einen Rezeptor $R_2$, der in fester Phase vorliegt und mit dem Rezeptor $R_1$ bindefähig ist, enthält, wobei der unlösliche Rezeptor $R_2$ und der lösliche Rezeptor $R_1$ physikalisch getrennt voneinander vorliegen, und wobei $R_1$ und $R_3$ jeweils das für den zu bestimmden Antikörper spezifische Epitop tragen.

Da der Rezeptor $R_3$ weder mit $R_1$ noch mit $R_2$ reagiert, ist seine Einbringung in das Reagenz unkritisch. Zweckmäßig werden jedoch $R_1$ und $R_3$ zusammen in einem Reagenz aufbewahrt.

Dabei sind $R_1$ und $R_3$ gemeinsam in einem flüssigen oder lyophilisierten Reagenz enthalten. $R_2$ liegt davon getrennt, an ein unlösliches Trägermaterial gebunden, vor.

In einer weiteren Ausführungsform können die Rezeptoren $R_1$ und $R_3$ löslich und Rezeptor $R_2$ unlöslich und getrennt von $R_1$ in einem festen Trägermaterial enthalten sein. Als Trägermaterial sind die unter den bei der Erfindung auftretenden Bedingungen inerten Materialien, welche $R_2$ durch chemische oder physikalische

4

Bindung zu fixieren vermögen, geeignet. Derartige Materialien sind dem Fachmann in großer Zahl bekannt. Bevorzugt ist ein saugfähiges, quellfähiges oder lösliches filmbildendes Trägermaterial, z. B. ein für Teststreifen bekanntes Trägermaterial wie Papier oder ähnliche Vliesmaterialien.

Die folgenden Beispiele und Zeichnungen erläutern die Erfindung weiter. Es zeigen:

Fig. 1    eine Eichkurve für ein Verfahren nach Beispiel 4 mit steigenden Mengen HBs-Antikörper

Fig. 2    eine Eichkurve für ein Verfahren nach Beispiel 5 mit steigenden Mengen HBs-Antikörper.

**Beispiel 1**

Herstellung von Reagenzlösungen

1) Herstellung eines HBsAg-Maus-IgG-Konjugates (Rezeptor-1)

1a Limitierte Reduktion von HBsAg (Hepatitis B-Oberflächen-Antigen)

5 mg HBsAg (Herstellung nach J. Virol. Meth. 11, 225-230 (1985)) in 5 ml 10 mM Na-Acetat-Puffer, pH 4,5 werden ad 50 mM Dithiothreit gebracht und 30 Minuten bei 25°C gehalten.

1b Entsalzung des Reaktionsansatzes

Der Reaktionsansatz wird über eine Sephadex® G-25 Säule (⌀ 16 mm, Packhöhe 15 cm) in 10 mM Na-Acetat-Puffer pH 4,5 mit einer Flußrate von 40 ml/h chromatographiert und fraktioniert. Die Fraktionen mit Absorptionen bei 280 nm (E 0,3) werden gepoolt und 24 Stunden gegen 30 mM K-Phosphatpuffer pH 7,1 dialysiert (3 x 1 l Dialyseflüssigkeit).

1c Modifizierung von IgG (Maus-normal-IgG, M-n IgG, Hersteller Calbiochem GmbH, Frankfurt). Zu einer Lösung von 10 mg M-nIgG in 1 ml 30 mM K-Phosphatpuffer pH 7,1 werden 10 µl einer Lösung von Maleinaminohexanoyl-N-hydroxy-succinimid-ester (MHS) in Dimethylsulfoxid (14,3 mg MHS/1 ml Dimethylsulfoxid) gegeben und 1 Stunde bei 25°C reagieren lassen.

1d Entsalzung des Modifizierungsansatzes

Der Ansatz aus 1c wird über 10 ml Sephadex® G 25 in 30 mM K-Phosphat-Puffer pH 7,1 chromatographiert und fraktioniert (Flußrate 40 ml/h). Die Fraktionen des Proteinpeaks mit der höchsten Proteinkonzentration werden gepoolt. Diese enthalten den Maleinaminohexanoyl-modifizierten M-nIgG Antikörper.

1e Konjugationsansatz

Zu 2 mg reduziertem HBsAg aus 1b in 4 ml 30 mM K-Phosphatpuffer pH 7,1 werden 10 mg nach 1d modifiziertes M-n-IgG in 1,6 ml 10 mM K-Phosphatpuffer pH 7,1 gegeben. Nach Mischen läßt man 1 Stunde bei 25°C reagieren. Die Reaktion wird beendet durch Zugabe von Cystein ad 1 mM, man läßt 30 Minuten bei 25°C reagieren, gibt dann Jodacetamid ad 5 mM zu und läßt 30 Minuten bei 25°C reagieren. Anschließend wird auf eine Konzentration von 15 mg/ml konzentriert.

1f Chromatographische Trennung des Konjugatansatzes

Der Ansatz wird nach Konzentrieren auf 0,8 ml auf Superose® 6 prep (Pharmacia) (ca. 80 ml) aufgetragen und mit einer linearen Fließrate von 5 cm/h chromatographiert (10 mM K-Phosphatpuffer pH 7,5; 0,2 % Saccharose; 0,1 % Azid). Die Fraktionen im Ausschluß sind das geeignete Produkt.

2) Schaf-anti-Maus-Fcγ (Rezeptor-2)

2a Schaf-anti-Maus-Fcγ-Antiserum wird wie folgt aufgereinigt:

Ein diesen Antikörper enthaltendes Serum wird ad 1,8 M mit Ammoniumsulfat versetzt. Das Präzipitat wird in 15 mM Na-Phosphatpuffer pH 7,0 enthaltend 50 mM Na-Chlorid aufgenommen. Die so erhaltene Lösung wird einer Passage über DEAE-Zellulose unterworfen.

2b Immunsorptive Aufreinigung

Das Schaf-anti-Maus-Fcγ-Reagenz aus 2a wird in PBS-Puffer (phosphate buffered saline) pH 7,5 im Verlauf von 2 Stunden über Sepherosil-gebundenes Maus-IgG geleitet (ca. 1 ml M-IgG-Sepherosil pro 50 mg Reagenz-Protein). Nach Auswaschen des nicht gebundenen Proteins wird der spezifisch gebundene Anteil mit 1 M Propionsäure eluiert. Das Produkt wird dialysiert und lyophilisiert.

3) Herstellung eines HBsAg-βGal-Konjugates (Rezeptor-3)

3a,b) Limitierte Reduktion und Entsalzung von HBsAg analog zu 1a,b)

3c) Modifizierung der β-Galactosidase (β-Gal)

Zu einer Lösung von 10 mg βGal in 1 ml 30 mM Kaliumphosphatpuffer pH 7,1 werden 50 µl Bis-Maleimido-Methyl-Ether (BMME) (47 mg BMME/ml wasserfreiem Dimethylsulfoxid) gegeben, gemischt und eine Stunde bei Raumtemperatur reagieren gelassen.

3d) Entsalzung der modifizierten βGal

Der Reaktionsansatz aus 3c) wird auf 4°C abgekühlt und bei dieser Temperatur über 10 ml Sephadex® G-25 in 30 mM Kaliumphosphatpuffer pH 7,1 bei einer Fließgeschwindigkeit von 40 ml/h chromatographiert und fraktioniert. Die Fraktionen mit dem höchsten Proteingehalt werden gepoolt (Proteinkonzentration 6 mg/ml): β-Gal-BMME.

3e) Konjugatansatz

Zu 2 mg reduziertem HBsAg aus 3b) in 4 ml 30 mM Kaliumphosphatpuffer pH 7,1 werden 10 mg βGal-BMME in 1,6 ml des gleichen Puffers gegeben und nach Mischen 1 Stunde reagieren gelassen.

Die Reaktion wird durch Zugabe von Cystein ad 1 mM beendet. Nach 30 Minuten bei 25°C wird Iodacetamid ad 5 mM zugegeben und ebenfalls 30 Minuten reagieren gelassen. Anschließend wird der Ansatz auf 15 mg/ml konzentriert.

3f) Chromatographische Auftrennung des Reaktionsansatzes

Der Ansatz nach 3e) wird auf eine 80 ml Superose® 6 prep-Säule aufgetragen und mit einer Flußrate von 5 cm/h chromatographiert (Puffer: 10 mM Kaliumphosphatpuffer pH 7,5, 0,2% Saccharose, 0,1% Azid). Die Fraktionen im Ausschluß stellen das geeignete Produkt dar.

**Beispiel 2**

Bestimmung von Anti-HBs

A) Herstellung von Substratpuffer

    70 mmol/l HEPES/NaOH pH 7,0
    154 mmol/l Natriumchlorid
    3 g/l Rinderserumalbumin
    5 mmol/l Chlorphenolrot-β-D-galactosid (hergestellt nach DE 33 45 748)
    2 g/l Tween® 20 (nichtionogenes Detergenz)

B) Herstellung von Reagenzträgern

1) Reagenzträger 1:

    40 µl einer Lösung, die pro Liter
    100 mmol Natriumphosphat pH 7,3 (37°C),
    2 mmol Magnesiumchlorid,
    9 g Natriumchlorid,
    5 g Rinderserumalbumin,
    10 mg HBsAg-IgG (Rezeptor-1-Lösung)
    250 U HBsAg-β-Galactosidase-Konjugat
    (Rezeptor-3-Lösung; die Aktivität wird bestimmt mit ortho-Nitrophenyl-β-Galactosid bei 37°C).
    enthält, wird auf ein Vlies aufgetropft, das aus kommerziellem Polyesterpapier besteht. Anschließend wird bei Raumtemperatur getrocknet.

2) Reagenzträger 2:

    Auf ein Zellulose-Vlies werden nach dem Bromcyan-Aktivierungsverfahren (DE-OS 1768512) Schaf-Antikörper gegen den Fcγ-Teil von Maus-Antikörpern (Rezeptor-2-Lösung) fixiert, wobei pro g Fasermaterial 10 µg Antikörper zur Fixierung angeboten werden. Durch Waschen wird ungekoppelter Antikörper entfernt und das Vlies schonend bei Raumtemperatur getrocknet.

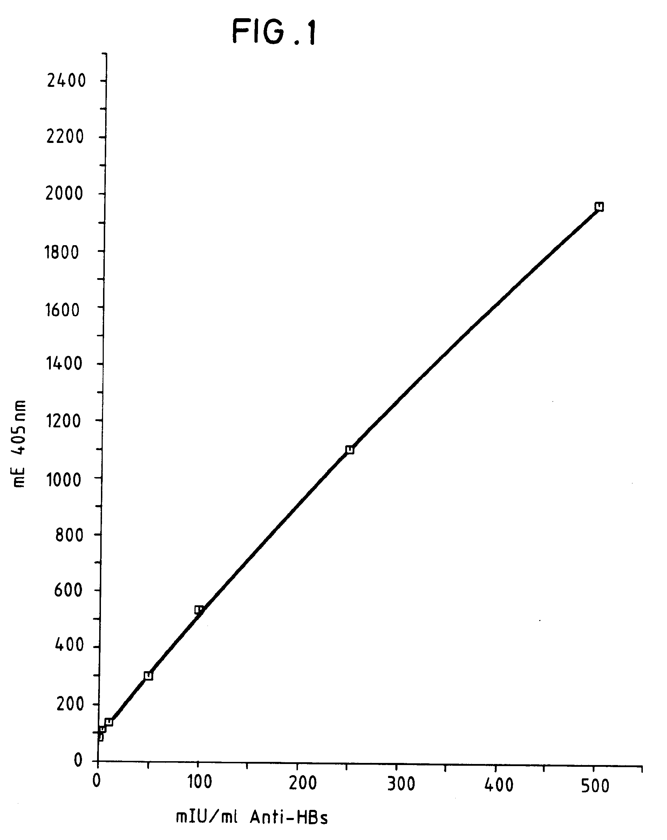

Die Bestimmung mit Hilfe dieser beiden Reagenzträger 1 und 2 erfolgt mit der in der DE-A1 3 425 008.5 beschriebenen Vorrichtung zur Durchführung analytischer Bestimmungen (vgl. dort Fig. 1).

Diese lehrt ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist. Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (Vk1) zu einer zweiten Ventilkammer (Vk2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (Vk2) verbunden ist. Fig. 1 der DE-A1 34 25 008.5 zeigt schematisch das verwendete Rotoreinsatzelement.

Reagenzträger 1 wird auf Feld c des Rotoreinsatzelements plaziert und Reagenzträger 2 auf Feld d. Dabei werden 40 µl Probe durch eine Öffnung am oberen Rand direkt auf das Feld a pipettiert. 270 µl Substratlösung werden in Kammer PK pipettiert. Durch ein geeignetes Zentrifugations-Programm, bei dem hohe Drehzahlen mit Stillstand abwechseln, werden dann Probe und Substratlösung in Richtung Trennmatrix und Küvette gefördert.

Dabei werden im Verlauf des Programms die Rezeptoren 1 und 3 durch die Probenflüssigkeit vom Feld c eluiert und die homogene Mischung anschließend zur Reaktion gebracht. Auf Feld d werden die gebildeten Komplexe (Rezeptor-1:Analyt:Rezeptor-3) an den Rezeptor 2 gebunden.

Die Substratlösung wird durch die Dosierkammer DK in Portionen geteilt, von denen die ersten zum Auswaschen von überschüssigem, nicht komplexiertem Konjugat dienen.

Unter diesen Bedingungen werden folgende Resultate erhalten:

## T a b e l l e    1

### Anti-HBsAg

| U/l[1] | 0 | 100 | 280 | 650 | 950 |
|---|---|---|---|---|---|
| Meßwert (mE) | 150+5 | 260+10 | 445+10 | 805+16 | 1111+27 |

[1] Definition der Einheiten entsprechend der Standardisierung des Paul-Ehrlich-Instituts, W.-Germany

Die Proben wurden jeweils 5fach bestimmt. Die Messungen wurden bei einer Schichtdicke von 0,3 cm bei 576 nm gegen 700 nm durchgeführt und auf eine Schichtdicke von d = 1 cm umgerechnet.

## Beispiel 3

Mikrotiterplatten (MTPs) werden pro Vertiefung mit je 250 µl einer Lösung von immunsorptiv aufgereinigtem Rezeptor 2 (Schaf-Anti-Maus-Fcγ-Antikörper) in der Konzentration von 10 µg/ml in Beschichtungspuffer (20 mM Carbonatpuffer, pH 9,6) über Nacht bei Raumtemperatur (RT) beschichtet. Im Anschluß werden unspezifische Bindungsstellen durch eine einstündige Nachinkubation bei Raumtemperatur mit 300 µl Nachbeschichtungspuffer (50 mM Kaliumphosphat, 150 mM NaCl, 1 % Rinderserumalbumin (RSA), 1 g/l Tween® 20, pH 7,5) abgesättigt. Nach Waschen mit 300 µl Waschpuffer (100 mM HEPES, 150 mM NaCl, 2,5 g/l Schafnormal-IgG, 0,5 mM Mg-L-Aspartat, 2 g/l Tween® 20, pH 7,25) pro Vertiefung werden 150 µl Probe und 50 µl einer Reagenzlösung, enthaltend Rezeptor 3 aus Beispiel 1 (HBsAg-βGal-Konjugat, 0,1 U/ml, Messung der Aktivität mit o-Nitrophenyl-β-D-Galaktosid bei 37°C) und Rezeptor 1 aus Beispiel 1 (HBsAg-Maus-IgG-Konjugat, 5 µg/ml) in Waschpuffer zugegeben, geschüttelt und anschließend für 3 Stunden inkubiert. Anschließend werden die Vertiefungen ausgesaugt und dreimal mit je 300 µl Waschpuffer gewaschen. Danach wird 1 Stunde bei Raumtemperatur mit 250 µl Substratlösung (5 mM Chlorphenolrot-β-D-Galaktosid, 70 mM HEPES, 150 mM NaCl, 3 g/l RSA, 2 g/l Tween® 20, pH 7,3) inkubiert und anschließend mittels eines handelsüblichen MTP-Meßgeräts bei 570 nm mit bichromatischer Korrektur bei 630 nm gemessen.

Tabelle 2 zeigt die Ergebnisse eines typischen Versuchs (Messungen jeweils mit n=4).

Tabelle   2

| Anti-HBsAg-Konzentration (U/l[1]) | 0 | 200 |
|---|---|---|
| mE (570nm-630nm) | 158+/-19 | 912+/-58 |

[1] Definition siehe Beispiel 2.

**Beispiel 4**

Beschichtung der Tubes

Polystyrol Testtubes werden 18 bis 24 Stunden mit 4 μg/ml Avidin in einem 40 mM Na-Phosphatpuffer bei Raumtemperatur beschichtet. Die Nachbeschichtung zur Absättigung unspezifischer Bindungsstellen erfolgt mit einer 0,3% RSA (Rinderserumalbumin), 0,9% NaCl, 2% Saccharose und 0,05% Polyetherglykol (Pluronic® L-64) enthaltenden Lösung.

Herstellung des mit Peroxydase konjugierten Hepatitis B Oberflächenantigens (HBsAg):

Das Verfahren wurde prinzipiell so gestaltet, daß durch schonende Reduktion im HBsAg SH-Gruppen freigesetzt werden, an die dann mit Maleimidohexanoyl-N-Hydroxy-Succinimidester (MHS) derivatisierte Meerrettich-Peroxydase (POD) gekoppelt wurde.

HBsAg wird bei einer Konzentration von 1,4 mg/ml und einem pH von 4,5 in Gegenwart von 10 mM Dithiothreit (DTT) 2 Stunden bei 25°C inkubiert und anschließend über eine Sephadex®-G25 fine-Säule (Pharmacia) in 10 mM Acetatpuffer entsalzt und zum Oxidationsschutz mit Argon begast.

POD wird bei einer Konzentration von 10 mg/ml in 30 mM Natriumphosphatpuffer, pH 7,1 mit einem 25fach molaren Überschuß an MHS für eine Stunde bei 25°C inkubiert. Freies MHS wird über eine Sephadex®-G25 medium Säule (Pharmacia) in 10 mM Kaliumphosphatpuffer, pH 6,1, 50 mM NaCl, 10 mM MgCl$_2$ abgetrennt.

Die Kopplung von reduziertem HBsAg an die POD erfolgt bei einem Einsatz von 3 mg MHS derivatisiertem POD/mg HBsAg bei pH 7 und 25°C. Nach 2 Stunden wird die Reaktion durch Zugabe von Cystein ad 1 mM gestoppt. Schließlich werden SH-Gruppen durch 5 mM Jodacetamid bei pH 7,5 1 Stunde bei 25°C derivatisiert. Das HBsAg-POD-Konjugat wird dann noch in 10 mM Natriumphosphatpuffer, pH 7,5, 150 mM NaCl über eine Gelfiltration an AcA22 (IBF, Serva, Germany) gereinigt.

Herstellung des biotinylierten Hepatitis B Oberflächenantigens:

Biotinyliert wird mit Biotin-N-Hydroxysuccinimidester (Bayer, E.A. und Wilchek M. (1980) Meth. Enzymol. 34, 265-267) über Aminogruppen im Protein:

Dazu wird HBsAg in 30 mM Natriumphosphatpuffer pH 7,1 bei einer Konzentration von 1 mg/ml eine Stunde bei 25°C mit 50 μl/ml einer 6 mg/ml-Lösung von Biotin-N-Hydroxysuccinimidester in DMSO inkubiert. Freies Biotinylierungsreagenz wird durch Gelfiltration an AcA 22 in 20 mM Natriumphosphatpuffer pH 7,1, 150 mM NaCl abgetrennt.

Durchführung des Tests:

Die Proben (200 μl, Serum oder Plasma) werden in den Tubes mit 1000 μl Reagenz bei Raumtemperatur 4 Stunden inkubiert. Das Reagenz enthält als Rezeptor R$_3$ 60 mU/ml mit Peroxydase konjugiertes Hepatitis B Oberflächenantigen (≙ 100 ng/ml HBsAg) und als Rezeptor R$_1$ 150 ng/ml biotinyliertes Hepatitis B Oberflächenantigen in Natriumphosphatpuffer pH 7,0 mit 0,5% Polyetherglykol (Pluronic® F-68), 0,2% Rinderserumalbumin, 0,2 M Na-Tartrat.

Nach erfolgter Inkubation werden die Tubes dreimal mit 1,5 ml Leitungswasser gewaschen und 1 ml 2,2'-

Azinodi/3-ethyl-benzthiazolin-sulfonsäure/diammoniumsalz (1,9 mM) für die Substratreaktion zupipettiert. Die Extinktion wird bei 405 nm in einer Küvette mit 5 mm Schichtdicke gemessen und auf eine 1 cm Küvette umgerechnet.

Tabelle 3 und Fig. 1 sind die Resultate eines typischen Versuchs zu entnehmen.

## T a b e l l e   3

|  | | Anti-HBs [1] | | | | | |
|---|---|---|---|---|---|---|---|
| U/l | 0 | 5,0 | 10 | 50 | 100 | 250 | 500 |
| Meßwert (mE) | 83 | 126 | 145 | 321 | 505 | 1110 | 1934 |

[1] Def. siehe Beispiel 2.

**Beispiel 5**

Es werden Testtubes mit einem Konjugat aus Streptavidin und Thermo-RSA beschichtet.

a) Herstellung von Thermo-RSA (Rinderserumalbumin):

1 g RSA-I werden in 100 ml 50 mmol/l Kaliumphosphat (pH 7,0) gelöst, auf 70°C erhitzt und 4 Stunden unter leichtem Rühren bei dieser Temperatur gehalten. Die Lösung wird abgekühlt, filtriert und in einer Ultrafiltrationszelle (Ausschlußgrenze: 30.000 Dalton) auf eine Konzentration von 50 mg/ml eingestellt. Anschließend wird gegen das 30fache Volumen an bidest. $H_2O$ dialysiert und anschließend lyophilisiert. Das Produkt hat ein Molekulargewicht von ca. 700.000.

Vor Kupplung an Streptavidin wird Thermo-RSA aktiviert. Hierzu werden 68 mg Thermo-RSA in 2 ml 0,1 mol/l Kaliumphosphat (pH 7,8) gelöst und langsam mit einer Lösung von 3,8 mg S-Acetylmercaptobernsteinsäureanhydrid (SAMBA) versetzt. Nach 3 Stunden Reaktionszeit wird gegen 2 1 50 mmol/l Kaliumphosphat (pH 6,5) bei 4°C dialysiert.

b) Herstellung von Streptavidin gekuppelt an Thermo-RSA

Aktivierung von Streptavidin:

60 mg Streptavidin werden in 6 ml 50 mmol/l Kaliumphosphat/100 mmol/l Natriumchlorid (pH 7,0) gelöst und bei 4°C kaltgestellt. 6,16 mg Maleinimidohexanoyl-N-hydroxysuccinimidester (MHS) werden in 620 µl Dioxan gelöst und in die Streptavidinlösung eingerührt. Nach einer Reaktionszeit von 2 Stunden bei 4°C wird zweimal gegen 1 l 50 mmol/l Kaliumphosphat/ 100 mmol/l Natriumchlorid (pH 5) bei 4°C dialysiert.

Herstellung eines Konjugats aus Streptavidin und Thermo-RSA:

66 mg MHS-derivatisiertes Streptavidin in 10 ml 50 mmol/l Kaliumphosphat pH 5,0 und 100 mmol/l Natriumchlorid gelöst und 72 mg aktiviertes Thermo-RSA-SAMBA in 5 ml 50 mmol/l Kaliumphosphat/100 mmol/l Natriumchlorid pH 6,5 werden zusammengegeben. Nach Vermischen werden 50 µl 1 mol/l Hydroxylamin pH 7,0 zugegeben, um die Reaktion zu stoppen. Nach 3 Stunden wird das Reaktionsproduct über Gelchromatographie (Superose® 6, 50 mmol/l Kaliumphosphat/100 mmol/l Natriumchlorid pH 7,5) aufgereinigt. Es wird ein Konjugat mit einem Molekulargewicht zwischen 1 und 5 Mio. erhalten.

In jedes Röhrchen Polystyrol bzw. Luran® (Hersteller BASF) werden 1,5 ml der Streptavidin/Thermo-RSA-Lösung (10 µg/ml Streptavidin/Thermo-RSA) gefüllt und über Nacht (ca. 22 Stunden) beladen. Danach werden die Röhrchen vollständig entleert und mit einer Rinderserumalbumin enthaltenden Lösung nachbeschichtet.

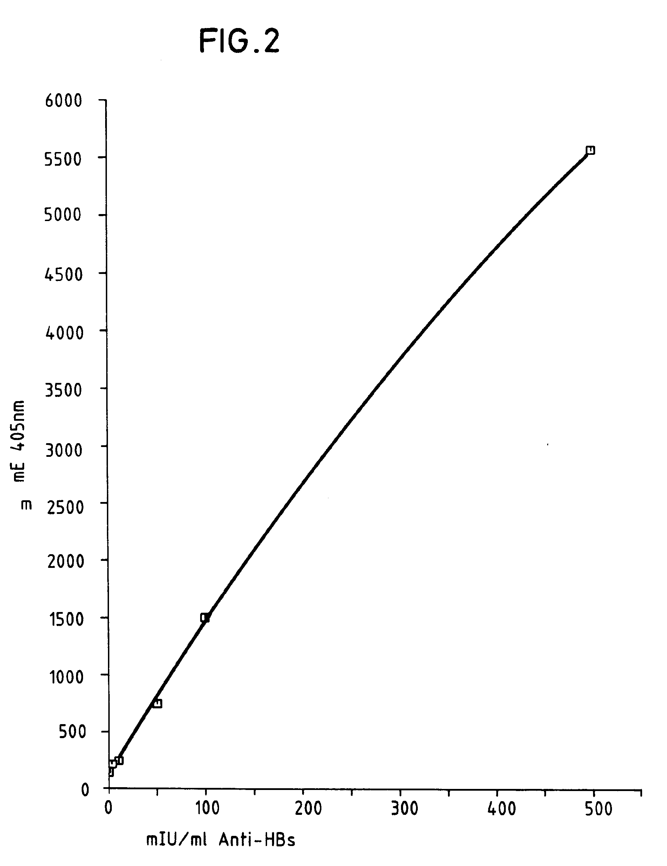

Es werden dann, wie im Beispiel 4 beschrieben, mit Peroxydase konjugiertes Hepatitis B Oberflächenantigen, biotinylierte Antigene hergestellt und, wie im Beispiel 4 beschrieben, ein Test durchgeführt. Die Resultate sind der folgenden Tabelle 4 und der Fig. 2 zu entnehmen.

EP 0 280 211 B1

<u>T a b e l l e   4</u>

Anti-HBs [1]

| U/l | 0 | 5 | 10 | 50 | 100 | 500 |
|---|---|---|---|---|---|---|
| Meßwert (mE) | 143 | 209 | 242 | 744 | 1500 | 5543 |

[1] Def. siehe Beispiel 2

**Patentansprüche**

1. Verfahren zur Bestimmung eines Antikörpers durch Inkubation mit drei verschiedenen Rezeptoren $R_1$, $R_2$ und $R_3$, von denen $R_1$ und $R_3$ in flüssiger Phase vorliegen und mit dem Antikörper bindefähig sind, $R_2$ an eine feste Phase gebunden vorliegt und mit $R_1$ bindefähig ist und $R_3$ eine Markierung trägt, Trennung der festen von der flüssigen Phase und Messung der Markierung in der festen Phase,
   **dadurch gekennzeichnet,**
   daß man als $R_1$ ein Konjugat aus einer vom zu bestimmenden Antikörper spezifisch erkannten Substanz und einem Reaktionspartner eines spezifischen Bindungssystems, als $R_3$ ein Konjugat aus einer vom zu bestimmenden Antikörper spezifisch erkannten Substanz und einer Markierung und als $R_2$ den anderen Bindungspartner des spezifischen Bindungssystems verwendet, wobei $R_1$ und $R_3$ jeweils das für den zu bestimmenden Antikörper spezifische Epitop tragen.

2. Verfahren nach Anspruch 1, **dadurch gekennzeichnet,** daß als spezifisches Bindungssystem Biotin/Avidin; Biotin/Streptavidin; Biotin/Antibiotin; Hapten/Antihapten; Fcγ-Fragment eines Antikörpers/Antikörper gegen dieses Fcγ-Fragment; Kohlehydrat/Lectin verwendet wird.

3. Verfahren nach Anspruch 1 oder 2,
   **dadurch gekennzeichnet,** daß der Rezeptor $R_3$ mit einem Enzym, einer fluoreszierenden, chemilumineszierenden oder radioaktiven Substanz markiert ist.

4. Verfahren nach einem der vorhergehenden Ansprüche,
   **dadurch gekennzeichnet,** daß man alle drei Rezeptoren gleichzeitig zusetzt.

5. Verfahren nach einem der Ansprüche 1 bis 3,
   **dadurch gekennzeichnet,** daß man die Rezeptoren $R_1$ und $R_3$ mit der Probe vorinkubiert und anschließend mit $R_2$ inkubiert.

6. Verfahren nach einem der vorhergehenden Ansprüche,
   **dadurch gekennzeichnet,** daß die Menge an vom zu bestimmenden Antikörper spezifisch erkannter Substanz in $R_1$ größer oder gleich der Menge an vom zu bestimmenden Antikörper spezifisch erkannter Substanz in $R_3$ ist.

7. Verfahren nach Anspruch 6, **dadurch gekennzeichnet,** daß das Verhältnis der vom zu bestimmenden Antikörper spezifisch erkannten Substanz in $R_1$ zu der vom zu bestimmenden Antikörper spezifisch erkannten Substanz in $R_3$ 20 bis 1:1 beträgt.

8. Verfahren nach Anspruch 7, **dadurch gekennzeichnet,** daß das Verhältnis 5 bis 1:1 beträgt.

9. Reagenz zur Bestimmung eines Antikörpers nach einem der vorhergehenden Ansprüche,
   **dadurch gekennzeichnet,** daß es zwei verschiedene lösliche, mit dem Antikörper bindefähige Rezepto-

ren $R_1$ und $R_3$, von denen $R_1$ einen Reaktionspartner eines spezifischen Bindungssystems und $R_3$ eine Markierung trägt und außerdem einen Rezeptor $R_2$, der in fester Phase vorliegt und mit dem Rezeptor $R_1$ bindefähig ist, enthält, wobei der unlösliche Rezeptor $R_2$ und der lösliche Rezeptor $R_1$ physikalisch getrennt voneinander vorliegen und wobei $R_1$ und $R_3$ jeweils das für den zu bestimmenden Antikörper spezifische Epitop tragen.

10. Reagenz nach Anspruch 9, **dadurch gekennzeichnet,** daß der Rezeptor $R_2$ bzw. der Rezeptor $R_1$ physikalisch getrennt voneinander jeweils auf einem Trägermaterial imprägniert vorliegen.

## Claims

1. Process for the determination of an antibody by incubation with three different receptors $R_1$, $R_2$ and $R_3$, of which $R_1$ and $R_3$ are present in liquid phase and are bindable with the antibody, $R_2$ is present bound to a solid phase and is bindable with $R_1$ and $R_3$ carries a labelling, separation of the solid from the liquid phase and measurement of the labelling in the solid phase, characterised in that as $R_1$ one uses a conjugate of a substance specifically recognised by the antibody to be determined and a reaction partner of a specific binding system, as $R_3$ a conjugate of a substance specifically recognised by the antibody to be determined and a labelling and as $R_2$ the other binding partner of the specific binding system, whereby $R_1$ and $R_3$ each carry the epitope specific for the antibody to be determined.

2. Process according to claim 1, characterised in that, as specific binding system, there is used biotin/avidin; biotin/streptavidin; biotin/antibiotin; hapten/antihapten; Fc$\gamma$ fragment of an antibody/antibody against this Fc$\gamma$ fragment; carbohydrate/lectin.

3. Process according to claim 1 or 2, characterised in that the receptor $R_3$ is labelled with an enzyme, a fluorescing, chemiluminescing or radio-active substance.

4. Process according to one of the preceding claims, characterised in that one adds all three receptors simultaneously.

5. Process according to one of claims 1 to 3, characterised in that one pre-incubates the receptors $R_1$ and $R_3$ with the sample and subsequently incubates with $R_2$.

6. Process according to one of the preceding claims, characterised in that the amount of substance in $R_1$ specifically recognised by the antibody to be determined is greater than or equal to the amount of the substance in $R_3$ specifically recognised by the antibody to be determined.

7. Process according to claim 6, characterised in that the ratio of the substance in $R_1$ specifically recognised by the antibody to be determined to the substance in $R_3$ specifically recognised by the anti-body to be determined amounts to 20 to 1:1.

8. Process according to claim 7, characterised in that the ratio amounts to 5 to 1:1.

9. Reagent for the determination of an antibody according to one of the preceding claims, characterised in that it contains two different soluble receptors $R_1$ and $R_3$ bindable with the antibody, of which $R_1$ carries a reaction partner of a specific binding system and $R_3$ carries a labelling and, furthermore, a receptor $R_2$ which is present in solid phase and is bindable with the receptor $R_1$, whereby the insoluble receptor $R_2$ and the soluble receptor $R_1$ are present physically separated from one another and whereby $R_1$ and $R_3$ each carry the epitope specific for the antibody to be determined.

10. Reagent according to claim 9, characterised in that the receptor $R_2$ or the receptor $R_1$, respectively, are present impregnated on a carrier material, each physically separated from one another.

## Revendications

1. Procédé de détermination d'un anticorps par incubation avec trois récepteurs différents $R_1$, $R_2$ et $R_3$ parmi lesquels $R_1$ et $R_3$ sont présents en phase liquide et sont capables de se lier avec l'anticorps, $R_2$ est lié à

une phase solide et est capable de se lier avec $R_1$, et $R_3$ porte un marqueur, séparation de la phase solide d'avec la phase liquide et mesure du marqueur dans la phase solide, caractérisé en ce que l'on utilise comme $R_1$ un conjugué d'une substance reconnue spécifiquement par l'anticorps à déterminer et d'un partenaire de réaction d'un système de liaison spécifique, comme $R_3$ un conjugué d'une substance reconnue spécifiquement par l'anticorps à déterminer et d'un marqueur et comme $R_2$ l'autre partenaire de liaison du système de liaison spécifique, $R_1$ et $R_3$ portant chacun l'épitope spécifique de l'anticorps à déterminer.

2. Procédé selon la revendication 1, caractérisé en ce que l'on utilise comme système de liaison spécifique biotine/avidine, biotine/streptavidine, biotine/antibiotine, haptène/antihaptène, fragment Fcγ d'un anticorps/anticorps contre ce fragment Fcγ, glucide/lectine.

3. Procédé selon la revendication 1 ou 2, caractérisé en ce que le récepteur $R_3$ est marqué avec une enzyme, ou une substance fluorescente, chimiluminescente ou radioactive.

4. Procédé selon l'une des revendications précédentes, caractérisé en ce que l'on ajoute simultanément les trois récepteurs.

5. Procédé selon l'une des revendications 1 à 3, caractérisé en ce que l'on pré-incube les récepteurs $R_1$ et $R_3$ avec l'échantillon puis on les incube avec $R_2$.

6. Procédé selon l'une des revendications précédentes, caractérisé en ce que la quantité de substance reconnue spécifiquement par l'anticorps à déterminer dans $R_1$ est supérieure ou égale à la quantité de substance reconnue spécifiquement par l'anticorps à déterminer dans $R_3$.

7. Procédé selon la revendication 6, caractérisé en ce que le rapport de la substance reconnue spécifiquement par l'anticorps à déterminer dans $R_1$ à la substance reconnue spécifiquement par l'anticorps à déterminer dans $R_3$ est de 20 à 1:1.

8. Procédé selon la revendication 7, caractérisé en ce que le rapport est de 5 à 1:1.

9. Réactif de détermination d'un anticorps selon l'une des revendications précédentes, caractérisé en ce qu'il contient deux récepteurs solubles $R_1$ et $R_3$ différents, capables de se lier avec l'anticorps, parmi lesquels $R_1$ porte un partenaire de réaction d'un système de liaison spécifique et $R_3$ porte un marqueur, et en outre un récepteur $R_2$ qui est présent en phase solide et qui est capable de se lier avec le récepteur $R_1$, le récepteur insoluble $R_2$ et le récepteur soluble $R_1$ étant présents séparés physiquement l'un de l'autre et $R_1$ et $R_3$ portant chacun l'épitope spécifique de l'anticorps à déterminer.

10. Réactif selon la revendication 9, caractérisé en ce que le récepteur $R_1$ et le récepteur $R_2$ sont présents physiquement séparés l'un de l'autre chacun sous une forme imprégnant un matériau support.

# FIG .1

# FIG.2